# EUROPEAN PATENT SPECIFICATION

(11) **EP 1 922 999 B1**
(45) Date of publication and mention of the grant of the patent: **03.08.2011**
(21) Application number: 06796522.8
(22) Date of filing: 18.08.2006
(51) Int. Cl.: A61B 6/00, A61B 5/00, G06T 1/00, G06T 7/00

(54) **IMAGE PROCESSING METHOD AND IMAGE PROCESSING DEVICE**
BILDVERARBEITUNGSVERFAHREN UND BILDVERARBEITUNGSEINRICHTUNG
PROCÉDÉ ET DISPOSITIF DE TRAITEMENT D'IMAGE

(30) Priority: 05.09.2005 JP 2005256385
(43) Date of publication of application: 21.05.2008
(73) Proprietor: Konica Minolta Medical & Graphic, Inc., Hino-shi, Tokyo 191-8511 (JP)
(72) Inventor: ISHIDA, Takayuki, Hiroshima 7370163 (JP); YANAGITA, Akiko, Tokyo 1630512 (JP); KAWASHITA, Ikuo, Hiroshima 7240613 (JP); YAMAMOTO, Megumi, Fukuoka 8110215 (JP); AKIYAMA, Mitoshi, Hiroshima7320066 (JP)
(74) Representative: Henkel, Feiler & Hänzel
(86) International application number: PCT/JP2006/316211
(87) International publication number: WO 2007/029467

(56) References cited:
- JP-A- 10 150 569
- JP-A- 10 255 035
- JP-A- 2005 185 560
- US-A- 6 084 981
- US-A1- 2005 100 208
- SUZUKI K ET AL: "NEURAL FILTER WITH SELECTION OF INPUT FEATURES AND ITS APPLICATION TO IMAGE QUALITY IMPROVEMENT OF MEDICAL IMAGE SEQUENCES" IEICE TRANSACTIONS ON INFORMATION AND SYSTEMS, INFORMATION & SYSTEMS SOCIETY, TOKYO, JP, vol. E85-D, no. 10, 1 October 2002 (2002-10-01), pages 1710-1718, XP001159312 ISSN: 0916-8532

## Description

### FIELD OF THE INVENTION

The present invention relates to an image processing method and image processing apparatus, wherein the output image with a specific pattern of an input image enhanced is outputted.

### BACKGROUND OF THE INVENTION

In one of the pattern recognition techniques known in the conventional art, a pattern is recognized by an discrimination device that has learnt a specific pattern having the characteristic shape, pattern, color, density and size, using the sampling data for learning known under the name of training data exemplified by the artificial neural network (hereinafter abbreviated as "ANN") or support vector machine.

In the medical field, this method is used to develop the apparatus for detecting a candidate area for the abnormal shadow by recognizing the pattern of the image area assumed to be the shadow (called the abnormal shadow) of a portion of lesion from the medical image obtained by examination of radiographing. This apparatus is called the CAD (Computer Aided Diagnosis Apparatus).

In common practice, when a discrimination device is used for pattern recognition, for example, preparation is made to get the pattern image of an abnormal shadow to be detected. Then image feature quantity including such statistical values as the average pixel value and distribution value or such geometric feature quantities as size and circularity in the image area of that abnormal shadow are inputted into the ANN as training data. Further, the ANN is made to learn in such a way that the output value close to "1" should be outputted if the pattern is similar to that of the abnormal shadow image. Likewise, using the pattern image of the shadow of a normal tissue (called the normal shadow), the ANN is made to learn in such a way that the output value close to "0" should be outputted if the pattern is similar to that of the normal shadow image. This arrangement ensures that, if the image feature quantity of the image to be detected is inputted to the aforementioned ANN, the output value of 0 through 1 is obtained from that image feature quantity. Accordingly, if this value is close to "1", it is highly likely that the shadow is abnormal; whereas, if this value is close to "0", it is highly likely that the shadow is normal. Thus, in the conventional CAD, the abnormal shadow candidates have been detected according to the output value obtained from this method.

In the aforementioned method, however, one training image (input value) corresponds to one output value. The output value heavily depends on the features of the specific pattern having been learnt, and therefore, this method is not powerful enough to discriminate the unlearned data. To improve the detection accuracy, a great number of specific patterns have to be learned.

One of the efforts to solve this problem is found in the development of the ANN technique (Patent Documents 1 and 2), wherein the image for pattern recognition is divided according to a predetermined area, the pixel value of each pixel within this area is inputted as the input value, and the indiscrete values of "0" through "1" representing the characteristics of the specific pattern are outputted as the pixel value of the pixel of interest located at the center of that area. In this technique, a predetermined pixel is compared with the features of the pixel constituting the specific pattern by using the information on the surrounding pixel. The ANN is made to learn so that the value close to "1" is outputted if the information is similar to the features of the pixel constituting the specific pattern and if not, the value close to "0" is outputted. To put it another way, an image having its specific pattern enhanced is formed by the output value from the ANN.

According to this method, the specific pattern of a predetermined pixel of interest including the information (pixel value) of the surrounding area thereof is learnt, and therefore a great number of input values and output values can be obtained from one training image. This method allows high-precision pattern recognition to be achieved by a small amount of training image. Further, there is an increase in the amount of information to be inputted into the discrimination device, with the result that the learning accuracy is improved.
Patent Document 1: U.S. Patent No. 6819790 Specification
Patent Document 2: U.S. Patent No. 6754380 Specification

SUZUKI K ET AL: "NEURAL FILTER WITH SELECTION OF INPUT FEATURES AND ITS APPLICATION TO IMAGE QUALITY IMPROVEMENT OF MEDICAL IMAGE SEQUENCES" IEICE TRANSACTIONS ON INFORMATION AND SYSTEMS, INFORMATION & SYSTEMS SOCIETY, TOKYO, JP, vol. E85-D, no. 10, 1 October 2002 (2002-10-01), pages 1710-1718, XP001159312 ISSN: 0916-8532 discloses neural filter with selection of input features and the application to image quality improvement of medical image sequences.

Furthermore, US2005/100208 A1 discloses an image modification and detection using massive training artificial neural networks.

### DISCLOSURE OF INVENTION

### PROBLEMS TO BE SOLVED BY THE PRESENT INVENTION

In the methods proposed in the aforementioned Patent Documents 1 and 2, however, there are few analytical factors in the discrimination device. Lots of procedures are hidden in a so-called black box; namely, there is no clear statement as to the influence under which the output value obtained from the discrimination device has been outputted from the input value. Thus, theoretical analysis is hardly possible. Accordingly, these methods will be restricted in terms of the degree of freedom in designing if they are to be put into practical use, for example, if they are to be adopted as CAD algorithms.

The object of the present invention is to solve the aforementioned problems and to provide an image processing method and an image processing apparatus characterized by excellent versatility, superb learning accuracy and a high degree of freedom in designing.

### MEANS FOR SOLVING THE PROBLEMS

According to the invention, the above object is achieved by an image processing method according to claim 1 and by an image processing apparatus according to claim 12. The dependent claims are directed to different advantages aspects of the invention.

The invention described in Structure (1) is an image processing method containing a learning step wherein a specific pattern is learned by a discrimination device using a training image having the aforementioned specific pattern and composed of the training input image to be inputted into the aforementioned discrimination device, and the training output image corresponding to the training input image, and an enhancement step wherein an enhanced image having the aforementioned specific pattern enhanced thereon is created from the image to be processed, by the discrimination device.

The invention described in Structure (2) is the image processing method described in Structure (1) wherein, in the aforementioned learning step, the pixel value of the pixel constituting the aforementioned training input image is inputted into the discrimination device, and the pixel value of the pixel constituting the aforementioned training output image is used as the learning target value of the discrimination device for the relevant input, whereby the aforementioned discrimination device learns.

The invention described in Structure (3) is the image processing method described in Structure (1) or (2) wherein the aforementioned training input image includes a plurality of training feature images created by applying image processing to the training input image, in the learning step, the pixel value of the pixel of interest located at the corresponding position in each of a plurality of the training input images is inputted into the discrimination device, and in the training output image, the pixel value of the pixel corresponding to the pixel of interest is set as the learning target value for the input of the discrimination device.

The invention described in Structure (4) is the image processing method described in Structure (3), wherein a plurality of the aforementioned training feature images are created in different image processing steps.

The invention described in Structure (5) is the image processing method described in Structure (4), wherein in the aforementioned enhancement step, a plurality of feature images are created by applying different image processing to the image to be processed, the pixel value of the pixel of interest located at the corresponding position in each of the image to be processed including a plurality of the aforementioned feature images is inputted into the discrimination device, and an enhanced image is structured in such a way that the output value outputted from the input value by the discrimination device is used as the pixel value of the pixel corresponding to the aforementioned pixel of interest.

The invention described in Structure (6) is the image processing method described in any one of Structures (1) through (5), wherein the training output image is an image created by processing the aforementioned training input image.

The invention described in Structure (7) is the image processing method described in any one of Structures (1) through (5), wherein the training output image is the pattern data formed by converting the specific pattern into a function.

The invention described in Structure (8) is the image processing method described in Structure (6) or (7) wherein the pixel value of the training output image is an indiscrete value.

The invention described in Structure (9) is the image processing method described in Structure (6) or (7) wherein the pixel value of the training output image is a discrete value.

The invention described in Structure (10) is the image processing method described in any one of Structures (3) through (5) wherein, in the aforementioned learning step, the training feature images are grouped according to the characteristics of the image processing applied to the training feature image, and the discrimination device learns according to the relevant group.

The invention described in Structure (11) is the image processing method described in any one of Structures (1) through (10), wherein the aforementioned training image is a medical image.

The invention described in Structure (12) is the image processing method described in Structure (11), wherein the training image is a partial image formed by partial extraction from a medical image.

The invention described in Structure (13) is the image processing method described in Structure (11) or (12), wherein the aforementioned specific pattern indicates an abnormal shadow.

The invention described in Structure (14) is the image processing method described in any one of Structures (1) through (13), further including a detection step wherein the aforementioned enhanced image is used to detect abnormal shadow candidates.

The invention described in Structure (15) is an image processing apparatus containing a learning device wherein a specific pattern is learned by a discrimination device using a training image having the aforementioned specific pattern and composed of the training input image to be inputted into the aforementioned discrimination device and the training output image corresponding to the training input image, and an enhancement device wherein an enhanced image having the aforementioned specific pattern enhanced thereon is created from the image to be processed, by the discrimination device.

The invention described in Structure (16) is the image processing apparatus described in Structure (15) wherein, in the aforementioned learning device, the pixel value of the pixel constituting the aforementioned training input image is inputted into the discrimination device, and the pixel value of the pixel constituting the aforementioned training output image is used as the learning target value of the discrimination device for the relevant input, whereby the aforementioned discrimination device learns.

The invention described in Structure (17) is the image processing apparatus described in Structure (15) or (16) wherein the aforementioned training input image includes a plurality of training feature images created by applying image processing to the training input image, the learning device ensures that the pixel value of the pixel of interest located at the corresponding position in each of a plurality of training input images is inputted into the discrimination device, and in the training output image, the pixel value of the pixel corresponding to the pixel of interest is set as the learning target value for the relevant input of the discrimination device.

The invention described in Structure (18) is the image processing apparatus described in Structure (17), wherein a plurality of the aforementioned training feature images are created in different image processing steps.

The invention described in Structure (19) is the image processing apparatus described in Structure (18), wherein a plurality of feature images is created by the aforementioned enhancement device by application of different image processing to the image to be processed, the pixel value of the pixel of interest located at the corresponding position in each of the image to be processed including a plurality of the aforementioned feature images is inputted into the discrimination device, and an enhanced image is structured in such a way that the output value outputted from the input value by the discrimination device is used as the pixel value of the pixel corresponding to the aforementioned pixel of interest.

The invention described in Structure (20) is the image processing apparatus described in any one of Structures (15) through (19), wherein the training output image is an image created by processing the aforementioned training input image.

The invention described in Structure (21) is the image processing apparatus described in any one of Structures (15) through (19), wherein the training output image is the pattern data formed by converting the specific pattern included in the training input image into a function.

The invention described in Structure (22) is the image processing apparatus described in Structure (20) or (21), wherein the pixel value of the training output image is an indiscrete value.

The invention described in Structure (23) is the image processing apparatus described in Structure (20) or (21) wherein the pixel value of the training output image is a discrete value.

The invention described in Structure (24) is the image processing apparatus described in any one of Structures (17) through (19) wherein, in the aforementioned learning device, the training feature images are grouped according to the characteristics of the image processing applied to the training feature image, and the discrimination device learns according to the relevant group.

The invention described in Structure (25) is the image processing apparatus described in any one of Structures (15) through (24), wherein the aforementioned training image is a medical image.

The invention described in Structure (26) is the image processing apparatus described in Structure (25), wherein the training image is a partial image formed by partial extraction from a medical image.

The invention described in Structure (27) is the image processing apparatus described in Structure (25) or (26), wherein the aforementioned specific pattern indicates an abnormal shadow.

The invention described in Structure (28) is the image processing apparatus described in any one of Structures (15) through (27), further including an abnormal shadow detecting device for detecting an abnormal shadow candidate by using the aforementioned enhanced image.

### EFFECTS OF THE INVENTION

According to the invention described in Structures (1) through (5) and (15) through (19), a great many input values (pixel value of the training feature image) and the output values (pixel value of the training output image) corresponding thereto can be obtained from one training input image. Further, the input values are accompanied by various forms of features, and therefore, multiple forms of pattern recognition can be performed. Thus, the learning accuracy of the discrimination device can be improved by a smaller number of training data items, and the pattern recognition performance of the discrimination device can be improved. Further, the pattern is enhanced and outputted by such a discrimination device, and easy detection of a specific pattern is ensured by the enhanced image. Moreover, the accuracy of the pattern recognition of the discrimination device can be adjusted by intentional selection of the training feature image to be used. This arrangement increases the degree of freedom in designing.

According to the invention described in Structures (6) through (9) and (20) through (23), the training output image can be created as desired, in response to the specific pattern required to be enhanced. This arrangement increases the degree of freedom in designing.

According to the invention described in Structures (10) and (24), the learning method of the discrimination device can be adjusted according to the group of image processing suitable for the pattern recognition of a specific pattern. This arrangement provides a discrimination device characterized by excellent sensitivity to a specific pattern.

According to the invention described in Structures (11) through (14) and (25) through (28), the doctor is assisted in detecting an abnormal shadow by the enhanced image wherein the abnormal shadow pattern is enhanced. When the enhanced image is used in the detection of abnormal shadow candidates, the false positive candidates can be deleted by the enhanced image in advance, with the result that the detection accuracy is improved.

### BRIEF DESCRIPTION OF THE DRAWINGS

Fig. 1 is a diagram showing the functional structure of an image processing apparatus in the present embodiment.
Fig. 2 is a flow chart illustrating a process of learning performed by the image processing apparatus.
Fig. 3 is a diagram showing an example of the training feature image.
Fig. 4 is a diagram showing examples of the training input image and training output image.
Fig. 5 is a diagram illustrating a process of learning by a discrimination device.
Fig. 6 is a diagram formed by plotting the normalized value of the training input image.
Fig. 7 is a diagram showing another example of the training output image.
Fig. 8 is a flow chart illustrating a process of enhancement performed by the image processing apparatus.
Fig. 9 is an example of creating an enhanced image from the image to be processed, by the discrimination device.
Fig. 10 is a diagram showing an example of the enhanced image.
Fig. 11 is a diagram showing an example of the enhanced image.
Fig. 12 is a diagram showing an example of the enhanced image.
Fig. 13 is a diagram showing a still further example of the enhanced image.
Fig. 14 is a diagram showing another structure example of the discrimination device.
Fig. 15 is a diagram illustrating pattern enhancement in group learning.
Fig. 16 is a diagram showing comparison between the results of enhancement processing by all-image learning and group image learning.

### DESCRIPTION OF REFERENCE NUMERALS

10. Image processing apparatus
11. Control section
12. Operation section
13. Display section
14. Communication section
15. Storing section
16. Image processing section
17. Abnormal shadow candidate detecting section
18. Learning data memory

### BEST MODE FOR CARRYING OUT THE INVENTION

The following describes an example of the case in the present embodiment wherein an abnormal shadow pattern is recognized as a specific pattern from the medical images by a discrimination device, and the enhanced image created by enhancement of this pattern is outputted. The specific pattern in the sense in which it is used here refers to the image having a characteristic shape, pattern, size, color, density and others.

In the first place, the structure will be described.

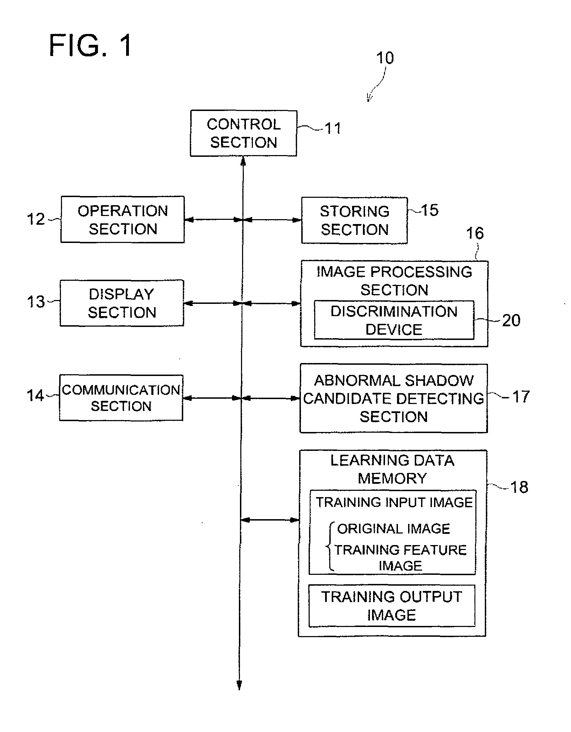

Fig. 1 shows the structure of the image processing apparatus 10 to which the present invention is applied.

The image processing apparatus 10 ensures that the enhanced image with the abnormal shadow pattern being enhanced is generated from the medical image obtained by examination radiographing, and detects the abnormal shadow candidate area from this enhanced image.

The abnormal shadow pattern in the sense in which it is used here refers to the image of the lesion appearing on the medical image. The abnormal shadow appears differently, depending on the type of the medical image and the type of the lesion. For example, the nodule as a type of medical findings of a lung cancer appears on the chest radiographic image as an approximately circular shadow pattern having a low density (white) and a certain magnitude. The abnormal shadow pattern often exhibits a characteristic shape, size, density distribution and others. Thus, distinction from other image areas can be made based on these characteristics in many cases.

This image processing apparatus 10 can be mounted on the medical image system connected through a network with various forms of apparatuses such as an image generation apparatus for generating an medical image, a server for storing and managing the medical image, and a radiograph interpreting terminal for calling up the medical image stored in the server for radiographic interpretation by a doctor and for displaying it on the display device. The present embodiment is described with reference to an example of implementing the present invention as the image processing apparatus 10 as a single system. However, it is also possible to make such arrangements that the functions of the image processing apparatus 10 are distributed over each of the components of the aforementioned medical image system so that the present invention is implemented as the entire medical image system.

The following describes the details of the image processing apparatus 10.

As shown in Fig. 1, the image processing apparatus 10 includes a control section 11, operation section 12, display section 13, communication section 14, storing section 15, image processing section 16, abnormal shadow candidate detecting section 17 and learning data memory 18.

The control section 11 contains a CPU (Central Processing Unit) and RAM (Random Access Memory). Reading out various forms of programs stored in the storing section 15, the control section 11 performs various forms of computation, and provides centralized control of processing in sections 12 through 18.

The operation section 12 has a keyboard and mouse. When the keyboard and mouse are operated by the operator, the operation signal in response to the operation is generated and is outputted to the control section 11. It is preferred to install a touch panel formed integrally with the display in the display section 13.

The display section 13 has a display device such as an LCD (Liquid Crystal Display). Various forms of operation screens, medical images and the enhanced images thereof are displayed on the display section in response to the instruction from the control section 11.

The communication section 14 is provided with the communication interface to exchange information with an external apparatus over the network. For example, the communication section 14 performs such communication operations as the operation of receiving the medical image generated by the image generation apparatus and the operation of sending, to a radiograph interpreting terminal, the enhanced image created in the image processing apparatus 10.

The storing section 15 stores the control program used in the control section 11; various processing programs for processing of learning and enhancement used in the image processing section 16 as well as the abnormal shadow candidate detection in the abnormal shadow candidate detecting section 17; parameters required to execute programs; and data representing the result of the aforementioned processing.

Through collaboration with processing program stored in the storing section 15, the image processing section 16 applies various forms of image processing (e.g., gradation conversion, sharpness adjustment and dynamic range compression) to the image to be processed. The image processing section 16 has a discrimination device 20. It executes the processing of learning and enhancement to be described later, so that a specific pattern is learned by the discrimination device 20 by the processing of learning. Then an enhanced image is created from the image to be process, in the processing of enhancement by the discrimination device 20 having learned.

The abnormal shadow candidate detecting section 17 applies processing of abnormal shadow candidate detection to the image to be processed, and outputs the result of detection. The enhanced image generated by processing of enhancement or the unprocessed medical image can be used as the image to be processed.

When the enhanced image is used as the processed image of the abnormal shadow candidate detecting section 17, the abnormal shadow is selectively enhanced in the enhanced image, and therefore, a relatively simple image processing technique such as the commonly known processing of threshold value or processing of labeling can be used in combination as the algorithm for abnormal shadow candidate detection processing. Further, a commonly known algorithm can be selected as desired, in response to the type of the abnormal shadow to be detected. In the breast image formed by radiographing the breasts, for example, abnormal shadows for a tumor, micro-calcified cluster and others are detected. In the case of the tumor, the shadow pattern exhibits a change in density of Gaussian distribution wherein the density decreases gradually toward the center in a circular form. Thus, based on such density characteristics, the abnormal shadow pattern of the tumor is detected from the breast image by a morphology filter or the like. In the meantime, the minute calcified cluster appears on the breast image as a collection of low-density shadows (clustered) exhibiting a change of density in an approximately conical form. So a triple ring filter or the like is employed to detect the abnormal shadow pattern having this density characteristic.

By way of an example, a triple ring filter will be described below. The triple ring filter is made up of three ring filters having a predetermined components of the intensity and direction of the density gradient created when a change in density exhibits an ideal conical form. In the first place, in the periphery of the pixel of interest, representative values for the components of the intensity and direction of the density gradient are obtained from the pixel value on each area of each ring filter. Based on the difference between the representative values and those for the components of the intensity and direction of the density gradient predetermined by each ring filter, the image area characterized by a change of density in an approximately conical form is detected as a candidate area.

The learning data memory 18 is a memory for storing the training data required for the learning by the discrimination device 20. The training data can be defined as the data required for the discrimination device 20 to learn a specific pattern. In the present embodiment, the training image including the abnormal shadow pattern is used as the training data. The training data is made up of a training input image which is inputted into the discrimination device 20, and a training output image corresponding to this training input image. These training images, together with the learning method for the discrimination device 20, will be discussed later.

The following describes the operation of the aforementioned image processing apparatus 10:

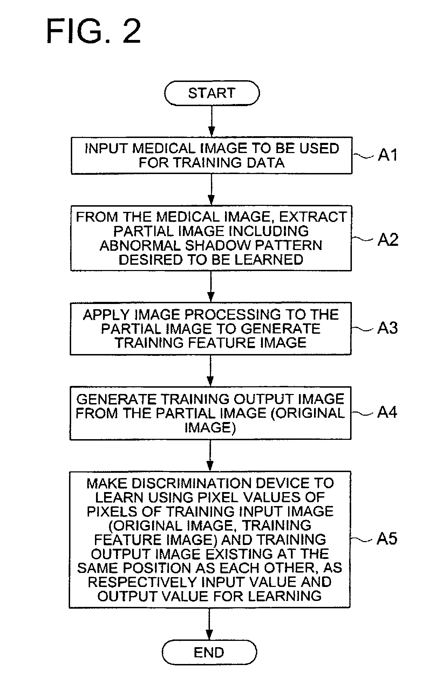

Referring to Fig. 2, the learning procedure for creating the discrimination device 20 will be described first. This learning procedure is executed when the image processing section 16 reads the learning procedure program stored in the storing section 15.

The following description refers to an example wherein the ANN is used as the discrimination device 20. Further, the following description assumes that the medical image including the abnormal shadow pattern is prepared for learning purposes in advance, and is stored in the learning data memory 18 as a training image.

In the learning procedure shown in Fig. 2, the medical image used as the training data is inputted (Step A1). To put it more specifically, in the image processing section 16, the medical image (training image) stored in the learning data memory 18 is read.

The specific pattern to be learned, namely, the partial image area including the abnormal shadow pattern is extracted from the inputted medical image (Step A2). In the following description, the partially extracted image will be referred to as the partial image. Extraction of the partial image is performed in such a way that, after radiographic interpretation of the training image, the doctor determines the area including the abnormal shadow pattern by visual observation, and this area is designated by the operation section 12. The image processing apparatus 10 extracts the image of the area corresponding to the designated area from the training image in response to this operation of designation. It should be noted that a plurality of partial images can be extracted from one training image.

Then plural different forms of image processing are applied to this partial image, and the training feature image is created (Step A3). The created training feature image is stored in the learning data memory 18. The training feature image is used as the training input image. To be more specific, the training input image contains the original medical image (hereinafter abbreviated as "original image" as distinguished from the training feature image) having been prepared for learning, and the training feature image. The training input image made up of the original image and training feature image is inputted into the discrimination device 20 so that it is used for training of learning by the discrimination device 20.

Primary differentiation and secondary differentiation for each of the directions X and Y can be mentioned as examples of abovementioned image processing. It is possible to apply the image processing using the primary differentiation filters such as Sobel filter and Prewitt filter, or the image processing that uses Laplacian filter and the eigen value of the Hessian matrix to produce the secondary differentiation-based feature. It is also possible to form an image of the calculated value or symbol of the curvature such as the average curvature or Gaussian curvature obtained for the density distribution curved surface of the aforementioned partial image, or to form an image of the quantity of the Shape Index or Curvedness defined according to the curvature. It is also possible to set a small area inside the aforementioned partial image, to calculate the average value while scanning the small area for each pixel (smoothening processing), or the statistics of the standard deviation inside the small area, median value and others, and to form an image of the results of these operations.
Further, it is also possible to create a frequency component image wherein the aforementioned partial image is separated into a plurality of frequency bands through the wavelet transformation and various forms of de-sharpening process.

Further, pre-processing can be applied prior to various forms of the aforementioned image processing. Pre-processing is exemplified by processing of the gradation transformation using the linear or non-linear gradation transformation characteristics, and by processing of the background trend correction which removes the density gradient from the background by means of polynomial approximation and band pass filter.

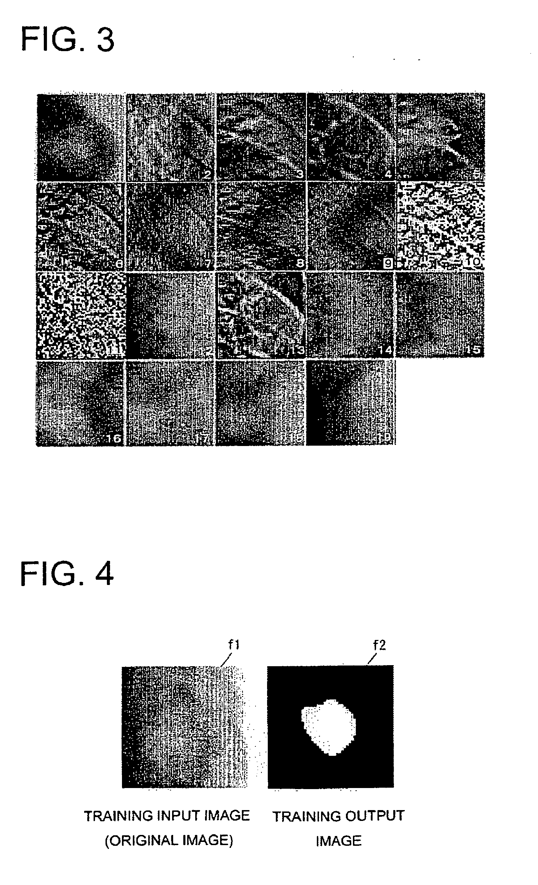

Fig. 3 shows an example of each training feature image resulting from the aforementioned image processing.

In Fig. 3, image 1 is an original image, and images 2 through 19 are training feature images having been subjected to various forms of image processing.

The training feature images 2 through 5 have been subjected to image processing corresponding to primary differentiation. Image 2 is a primarily differentiated image in the x-axis direction; image 3 is a primarily differentiated image in the y-axis direction; image 4 is a Sovel filter output (edge enhancement); and image 5 is a Sovel filter output (edge angle). The training feature images 6 through 9 have been subjected to image processing corresponding to secondary differentiation. Image 6 is a Laplacian filter output; image 7 is a secondarily differentiated image in the x-axis direction; image 8 is a secondarily differentiated image in the y-axis direction; and image 9 is a secondarily differentiated image in the x- and y-axis directions. The training feature images 10 and 11 represent the images wherein the curvatures are converted into codes. The image 10 is the image formed by converting the average curvature into a code, and the image 10 is the image formed by converting the Gaussian curvature into a code. The image 12 is a smoothened image (3 x 3); image 13 is a standard deviation image (3 x 3). The training feature images 14 through 19 indicate the images classified according to frequency component by wavelet transformation. The image 14 is the high frequency component image of the wavelet (Levels 1 through 3), the image 15 is the high frequency component image of the wavelet (Levels 2 through 4), the image 16 is the intermediate frequency component image of the wavelet (Levels 3 through 5), the image 17 is the intermediate frequency component image of the wavelet (Levels 4 through 6), the image 18 is the low frequency component image of the wavelet (Levels 5 through 7), and the image 19 is the low frequency component image of the wavelet (Levels 6 through 8). In this manner, the training feature images 2 through 19 can be classified into groups of similar property according to the characteristics of image processing.

The step of creating the training feature images is followed by the step of producing the training output images (Step A4). The training output image is the image that provides a learning target for the input of the training input image into the discrimination device 20.

Fig. 4 shows the examples of a training input image and a training output image.

The training output image f2 is produced to correspond to the training input image (original image) denoted by reference numeral f1. It shows the examples of the training output images produced by artificial processing through binarization wherein the area corresponding to the abnormal shadow pattern is assigned with "1", and other areas are assigned with "0". The area pertaining to the abnormal shadow pattern is designated through the operation section 12 by the doctor evaluating this area in the training input image f1. In response to this operation of designation, the image processing apparatus 10 creates the training output image wherein the pixel value of the designated area is set to "0", and that for other area is set to "1".

When the training input image and training output image have been produced in the aforementioned manner, they are used for learning by the discrimination device 20.

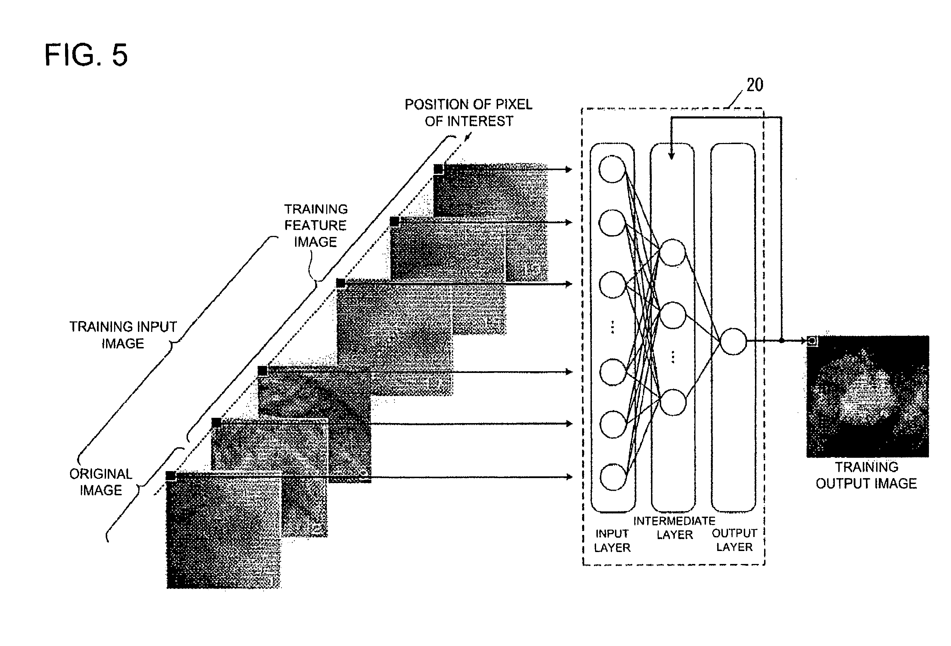

The discrimination device 20 is a hierarchical ANN, as shown in Fig. 5. The hierarchical ANN is formed of an input layer made up of input neuron that receives the input signal and distributes it to other neuron, an output layer made up of the output neuron that outputs the output signal to the outside, and an intermediate layer made up of a neuron that lies between the input neuron and output neuron. The neuron of the intermediate layer binds all neurons of the input layer and the neuron of the output layer binds all neurons of the intermediate layer.

The neuron of the input layer binds only with the neuron of the intermediate layer, and the neuron of the intermediate layer binds only with the neuron of the output layer. This arrangement allows the signal to flow from the input layer to the intermediate layer, then to the output layer. In the input layer, the input signal having been received is outputted directly to the neuron of the intermediate layer, without processing of the signal by neuron. In the intermediate and output layers, signal processing is carried out, for example, the signal inputted from the previous layer is assigned with weights by the bias function set on the neuron, and the processed signal is outputted to the neuron of the subsequent layer.

When the discrimination device 20 learns, the pixel of interest is set to the training input image (original image), and the pixel value of this pixel of interest is obtained. Further, in a plurality of training feature images and the training output image, the pixel value of the pixel corresponding to the pixel of interest of the original image is obtained. Each pixel value obtained from the original image and training feature image is inputted in discrimination device 20 as an input value and the pixel value obtained from training output image is set to the discrimination device 20 as the target value for learning. The learning of the discrimination device 20 is carried out in such a way that the value close to the target value for learning will be outputted from this input value (step A5).

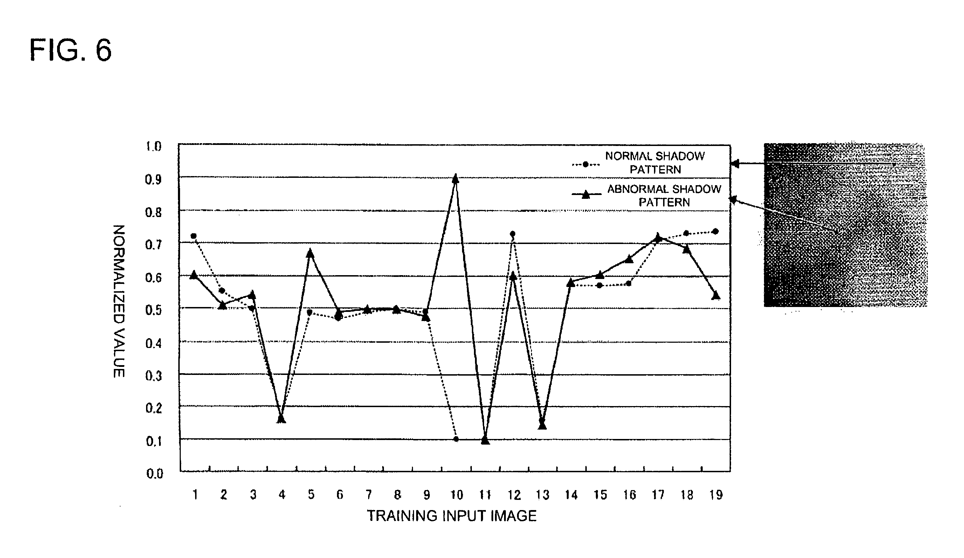

The pixel value is used as the input value to the discrimination device 20 after having been normalized to the value 0 through 1, so that the standard of the input values of training input images having different features are normalized to the same level. Fig. 6 is formed by plotting the values obtained by normalizing the pixel value in a certain pixel in the training input image (original image 1 and training feature images 2 through 19 in Fig. 3). In Fig. 6, the normalized values connected by dotted lines indicate the values obtained by normalizing the pixel value constituting the image pattern of the normal tissue (hereinafter abbreviated as "normal shadow pattern") in the training input image. The normalized values connected by solid lines indicate the normalized pixel value of the pixel constituting the abnormal shadow pattern.

When the discrimination device 20 learns, the output value gained from the discrimination device 20 by inputting the pixel values of the training input images into the discrimination device 20 is compared with the pixel value gained from the training output image, as shown in Fig. 5, and the error thereof is calculated. In this case, the output values outputted from the discrimination device 20 are indiscrete values of 0 through 1. Then the parameter of the bias function in the intermediate layer is optimized so that the error will be reduced. The error back propagation method can be used as a method of learning to achieve optimization for example. To be more specific, when the parameter is reset by optimization, the pixel value gained from the training feature image is again inputted into the discrimination device 20. Optimization of the parameter is repeated many times in such a way as to minimize the error between the outputted value obtained from the input value and the pixel value of the training output image, whereby the learning of the abnormal shadow pattern is achieved.

Upon completion of learning of one pixel of interest, the position of the pixel of interest is shifted the distance corresponding to one pixel in the direction of main scanning on the original image. Then the same learning procedure is repeated for the newly set pixel of interest. In this manner, the pixel of interest is scanned in the directions of main scanning and sub-scanning of the training input image. Upon completion of learning for all the pixels of the training input image, the discrimination device 20 having completed learning of the abnormal shadow pattern is provided.

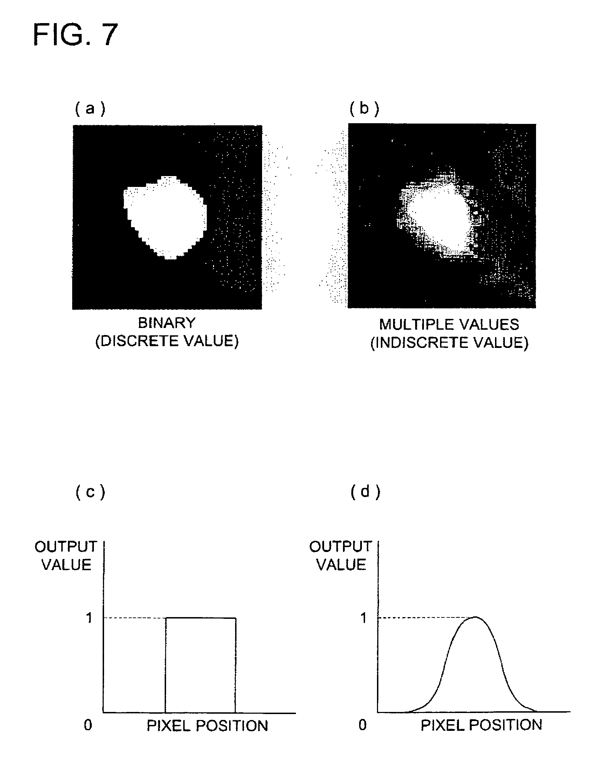

The training output image is not restricted to the binary value (discrete value) shown in Fig. 7 (a). It is also possible to create a multivalued image (indiscrete value), as shown in Fig. 7 (b). The multivalued image can be produced by de-sharpening the binary image of Fig. 7 (a) which is created in advance.

It is also possible to produce the pattern data wherein the abnormal shadow pattern is not the image but is formed into a function. To be more specific; it is possible to produce the pattern data (Fig. 7 (c) and 7 (d)) wherein the output value corresponding to each of the pixel position is set. The pattern data shown in Fig. 7 (c) shows the data obtained by using a discrete value as the output value. It indicates the output value (vertical axis) of "0" or "1" set in response to the pixel position (horizontal axis). In the meantime, the pattern data of Fig. 7 (d) is obtained by using an indiscrete value as the output value. It indicates the output value of "0" through "1" set in response to the pixel position. It should be noted that Figs. 7 (c) and 7 (d) show the setting data for one line. In actual practice, the setting data of such an output value is set two-dimensionally in response to the pixel position in the directions of main scanning and sub-scanning.

When the discrete value is used to represent the output value of the pattern data, it is expected to obtain the effect of forcibly increasing the degree of the enhancement within the area of the abnormal shadow pattern of enhanced image. In the meantime, the indiscrete value is used to represent the pattern data output value, a change in the output value from the center toward the circumference of the shadow pattern exhibits Gaussian distribution. This arrangement can be expected to meet the requirements even if the size of the abnormal shadow pattern is different from that of the learnt one to some extent. The same thing can be said for the cases of using the image shown in Figs. 7 (a) and 7 (b).

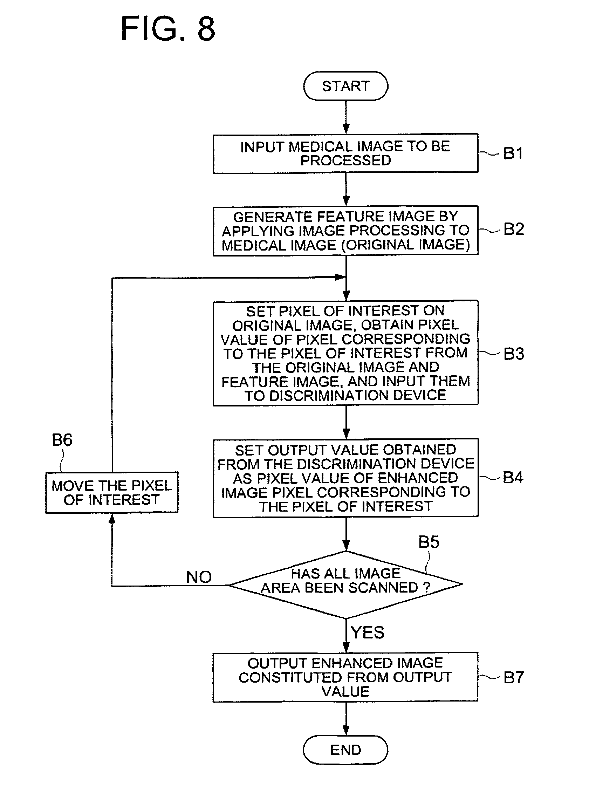

Referring to Fig. 8, the following describes the processing of enhancement for creating an enhanced image from the medical image to be processed, by the discrimination device 20 having completed the step of learning. Similarly to the case of learning, the processing of enhancement is executed by the collaboration with the enhancement processing program stored in the image processing section 16 and storing section 15.

In the processing of enhancement in Fig. 8, the medical image to be enhanced is inputted in the first place (Step B1). To be more specific, the medical image to be processed stored in the storing section 15 is read out by the image processing section 16. This is followed by the step of applying different image processing to the medical image, whereby a plurality of feature images are created (Step B2). The image processing to be applied in this case is the same form of image processing which is applied when creating the training feature image, and is applied under the same conditions as well.

When the feature image has been created, the pixel of interest is set to the original medical image (referred to as "original image" as distinguished from the feature image), and the pixel value of this pixel of interest is obtained. Further, in the feature image, the pixel value of the pixel located at the position corresponding to that of the pixel of interest is obtained. The pixel values obtained from the original image and the feature image are normalized to values 0 through 1 to produce the normalized value, which is then inputted into the discrimination device 20 (Step B3). When the output value has been obtained from the discrimination device 20 through this input value, the output value is set as the pixel value of the pixel that constitutes the enhanced image (Step B4).

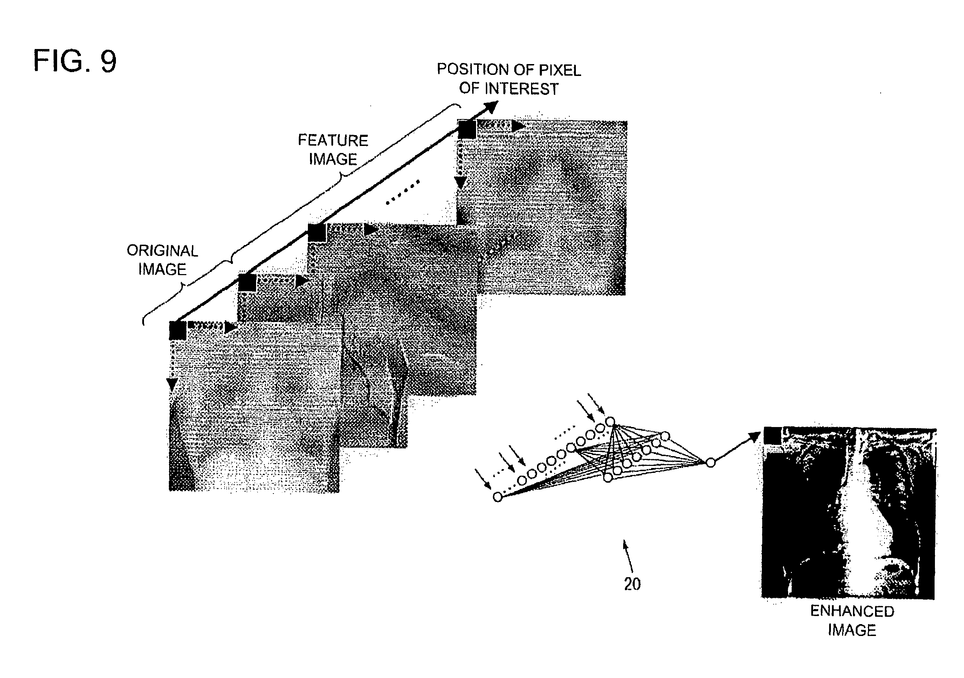

Fig. 9 shows the relationship between the input value and output value of the discrimination device 20.

As shown in Fig. 9, in the enhanced image, the output value from the discrimination device 20 is set at the pixel value of the pixel corresponding to the position of the pixel of interest set on the original image.

In this manner, when the output value corresponding to one pixel has been gained from the image to be processed, by the discrimination device 20, the pixels of interest are set in all image areas, and a decision is made to see whether scanning has been completed or not (Step B5). If scanning has not been completed (Step B5: N), the position of the pixel of interest is shifted the distance corresponding to one pixel in the direction of main scanning on the original image (Step B6), and the processing of Steps B3 and B4 is repeatedly applied to the pixel of interest newly set by this shift.

When the pixel of interest has been scanned for all the image areas (in the directions of main scanning and sub-scanning) (Step B5: Y), the enhanced image formed so that the output value from the discrimination device 20 is used as the pixel value is outputted (Step B7).

The output value from the discrimination device 20 is outputted as the indiscrete value of "0" through "1". When the enhanced image is outputted to the display apparatus or film, the output value is outputted after having been converted into the luminance level or density level according to the requirements of the output device. When the value is converted into the luminance value, the output values of "0" through "1" are assigned to Kₘᵢₙ through Kₘₐₓ, assuming that the output value "0" is the minimum luminance level Kₘᵢₙ (black when displayed), and the output value "1" is the maximum luminance level Kₘₐₓ (white when displayed). In the meantime, when the value is converted into the density value, the output values of "0" through "1" are assigned to Dₘᵢₙ through Dₘₐₓ, assuming that the output value "0" is the minimum density level Dₘᵢₙ (black on the film), and the output value "1" is the maximum density level Dₘₐₓ (white on the film).

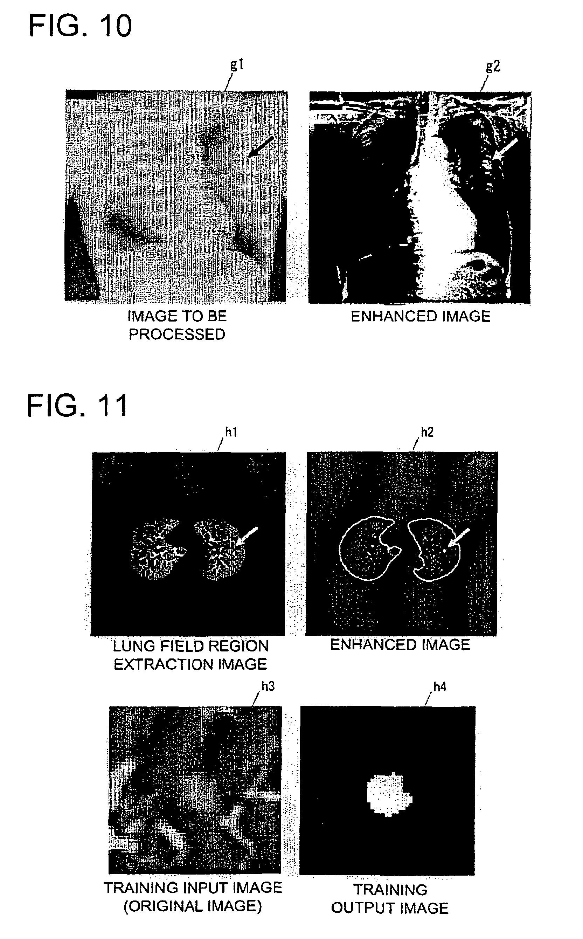

Fig. 10 shows an example of the enhanced image.

The image g1 to be processed on the left of Fig. 10 is a breast X-ray image (original image). When this image g1 was applied to the discrimination device 20, the enhanced image g2 on the right was outputted. Although, in the image g1 to be processed, an abnormal shadow pattern is located at the arrow-marked position, its discrimination is difficult on the image g1 to be processed. In the image g2 to be processed, however, the abnormal shadow pattern is clearly marked by a round pattern of low density. This shows that this area is more enhanced than other image areas.

In the example of the enhanced image h2 shown in Fig. 11, the partial image h3 including the abnormal shadow pattern (shadow area indicated by arrow) is extracted as a training input image from the image h1 to be processed in the breast CT image, and the training output image h4 is created from this partial image h3. This is used for learning by the discrimination device 20. Then the enhanced image h2 is created from the image h1 to be processed by the discrimination device 20 having learnt. It should be noted that the image h1 to be processed is the image wherein only the lung field region is extracted by image processing. This image h1 to be processed includes many normal shadow patterns including blood vessels that are likely to be confused with the abnormal shadow of the nodule. In the enhanced image h2, the characteristics of these normal shadow patterns are reduced and only the abnormal shadow patterns are successfully enhanced, as can be observed.

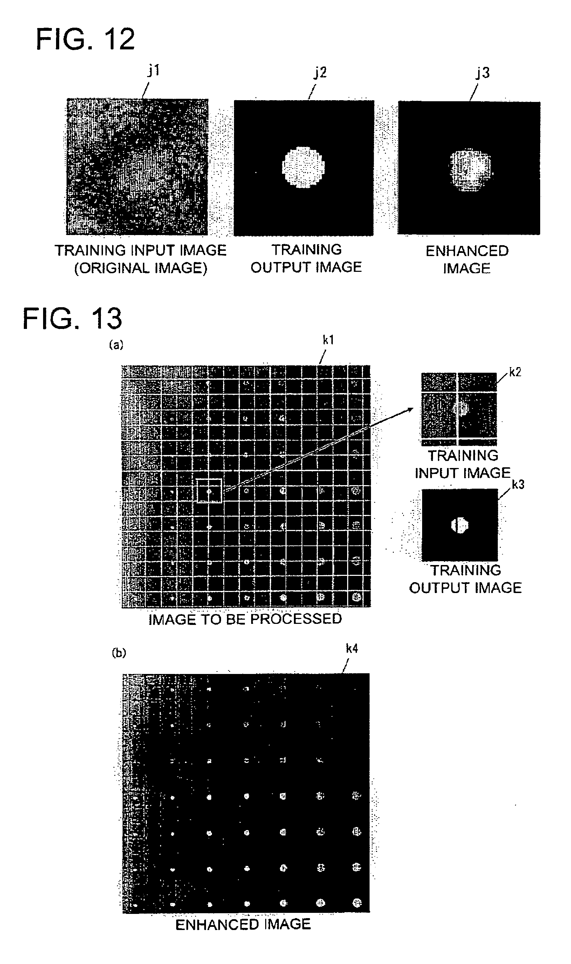

As shown in Fig. 12, an image j1 to be processed characterized by low image quality and coarse granularity was prepared. Then a training output image j2 exhibiting the abnormal shadow pattern was created from the image j1 to be processed, whereby learning of the discrimination device 20 was performed. Then the image j1 to be processed was again inputted into the discrimination device 20 having learnt. This resulted in outputting of the enhanced image j3 as shown in Fig. 12. As is apparent from the enhanced image j3, the noise which had been conspicuous in the image j1 to be processed was reduced, and only the abnormal shadow pattern was enhanced.

As shown in Fig. 13 (a), the simulated circular pattern of low density was changed in size and contrast, and a plurality of resulting patterns were set to the test object k1, to which the discrimination device 20 of the present embodiment was applied. The test object k1 is provided with a lattice pattern of low density in addition to the simulated patterns. The learning of the discrimination device 20 was conducted by creating the training output image k3 corresponding thereto, wherein a desired simulated pattern of the test object was used as a training input image k2. The training output image k3 having been created was binary. This results in formation of the enhanced image k4 shown in Fig. 13 (b). As shown in Fig. 13 (b), the discrimination device 20 mitigates the features of the lattice pattern and allows only the simulated patterns to be enhanced.
Further, in each of the simulated patterns in the test object k1, even when there is an overlap of lattice patterns in the form different from that of the lattice pattern included in the training input image k2, it is possible to enhance the image area if it has the same features as those of the simulated pattern contained in the training input image k2, as can be observed. Further, all the simulated patterns of any size are enhanced on the enhanced image k4, and it can be seen that it is possible to meet requirements in the size of the pattern to be enhanced, to some extent.

After processing of enhancement, the enhanced image having been created is outputted to the abnormal shadow candidate detecting section 17 from the image processing section 16. The detection of the abnormal shadow candidate is started by the abnormal shadow candidate detecting section 17. When the abnormal shadow pattern has been detected from the enhanced image, information on the abnormal shadow candidate (e.g., a marker image for the arrow mark or the like that indicates the position of the abnormal shadow candidate area) is displayed on the display section 13 as the doctor's diagnosis assisting information.

The enhanced image having been created can be simply used by the doctor for radiographic interpretation.

As described above, according to the present embodiment, the training feature image is formed by applying various forms of image processing to the original image, in addition to the original image, as the training input image. Use of this training feature image allows multiple input values to be gained from one image. In the conventional art, many of the discrimination devices 20 were so designed as to output the possibility of being abnormal shadow using the image feature quantity of a certain training image as an input value. According to this method, one output value corresponded to one input image, and therefore, the pattern could be recognized only when the features of the image to be processed were the same as those of the training image (abnormal shadow pattern). To meet the requirements of various forms of abnormal shadow patterns containing unlearnt data, a great number of training images had to be prepared. However, according to the present embodiment, a plurality of images are formed from one image, and further, the pixel values thereof are inputted into the discrimination device 20. Thus, a great number of input values and the output values corresponding thereto can be obtained from one image for learning. Further, these input values are provided with various forms of features so that learning can be made multilaterally. Thus, a small amount of data improves the learning accuracy of the discrimination device 20, and enhances the pattern recognition capacity of the discrimination device 20.

Further, the aforementioned discrimination device 20 produces the enhanced image wherein the abnormal shadow pattern is enhance. When this enhanced image is used to detect the abnormal shadow candidate or is employed for radiographic interpretation by the doctor, detection of the abnormal shadows is facilitated, whereby a significant contribution is made to assist the doctor's diagnosis.

Further, the pixel value obtained from the training feature image provided with various forms of image processing, namely, various forms of feature quantity can be utilized in the learning of the discrimination device 20. To put it another way, multifaced pattern learning can be achieved. Thus, this arrangement improves the pattern recognition accuracy of the discrimination device 20.

Further, some patterns can be more easily recognized by the features of image processing. The pattern recognition accuracy can be adjusting by selecting the type of the training feature image having been subjected to different image processing at the time of learning. Accordingly, when the training feature image (image processing applied to the original image) to be used is selected intentionally in response to the abnormal shadow pattern to be detected, an enhanced image can be formed for a specific abnormal shadow pattern alone. This arrangement enhances the degree of freedom in the design of the discrimination device 20.

It should be noted that, if the processing of enhancement for enhancing the abnormal shadow pattern is applied in a pre-processing step of abnormal shadow candidate detection, and the abnormal shadow pattern is enhanced in advance, then the features of the normal shadow pattern are lost. This arrangement substantially reduces the number of the falsely positive candidates (candidate less likely to be a abnormal shadow) to be detected, as compared to the case wherein the original medical image (original image) is used to detect the abnormal shadow candidate. Thus, this arrangement improves the accuracy of detecting the abnormal shadow candidate.

The embodiment of the present invention described so far represents a preferred example to which the present invention is applied. It is to be expressly understood, however, that the present invention is not restricted thereto.

For example, the aforementioned embodiment was explained with reference to an example of using the ANN as the discrimination device 20. However, it is also possible to use any discrimination device if it is capable of pattern recognition by pattern learning based on the training data, as exemplified by as a discrimination device based on the discrimination/analysis method and fuzzy inference, and a support vector machine. It should be noted that, for a technique of grouping into two classes as exemplified by Mahalanobis' distance, the output value given from the discrimination device 20 is binary.

The present embodiment has been described with reference to the example of detecting the abnormal shadow pattern included in the medical image. Without being restricted thereto, for example, the present invention can be applied to the processing of segmentation (region extraction) wherein pattern recognition of a particular region is performed, as exemplified by the case of extracting the lung field region from the medical image obtained by radiographing the breast. The present invention can also be used for pattern classification, e.g., for classification of interstitial shadow patterns included in a medical image created by radiographing a breast.

In the aforementioned embodiment, an example of using two-dimensional image processing was used for explanation. The three dimensional image processing can also be applied in the similar manner.

The above description of the embodiment referred to an example of applying the processing of detecting the abnormal shadow candidate in the step of detection after the enhanced image has been created in the step of enhancement. It is also possible to make such arrangements that, after the abnormal shadow candidates have been detected by the commonly known detection algorithm from the unprocessed medical image, they are distinguished between the truly positive candidate (true abnormal shadow candidate) and falsely positive candidate (less likely to be abnormal shadow candidate) in the step of discrimination. In this step of discrimination, the pattern recognition of the present invention is used to perform hereby the final abnormal shadow candidate is detected.

The present invention can be applied to other images in addition to the medical image alone when a specific pattern is to be enhanced from the image.

In the aforementioned example, it is intended to enhance a relatively small pattern such as an abnormal shadow. Accordingly, the partial image obtained by extracting an image partially from the medical image is used as the training image. Without the present invention being restricted thereto, the entire medical image can be used as the training image according to the particular requirement. For example, when the present invention is used for segmentation, relatively large regions of organs and others are often extracted. This does not require partial extraction of the image. In this case, the entire medical image should be used as the training image.

In the aforementioned embodiment, the number of the neurons on the output layer is one, namely, the number of the training output images used in the step of learning is one for the partial image. However, the number of the neurons on the output layer can be two or more; to put it another way, a plurality of training output images can be utilized.

For example, in the application to pattern classification, when the shadows contained in the image are to be classified into three specific patterns A, B and C, the training output image effective for enhancement of the pattern A, the training output image effective for enhancement of the pattern B and the training output image effective for enhancement of the pattern C are created as the training output images. They are correlated with three neurons on the output layer, whereby learning is performed. In the step of enhancement, one image to be processed is inputted, whereby three enhanced images are outputted. An enhanced image having the highest degree of enhancement is selected from among them, and the type of the pattern corresponding to that enhanced image is assumed as the result of classification. As an indicator showing the degree of enhancement, it is possible to use the statistical quantity such as the average of the pixel values of the enhanced image, and pixel value that provides a predetermined cumulative histogram value, for example.

The training feature image contains the pattern that can be easily recognized according to the characteristics of the image processing. Accordingly, the training feature images can be separated into groups according to the characteristics of the image processing, and the learning of the discrimination device can be conducted according to the group.

For example, in the example of the training feature image shown in Fig. 3, the images 1 through 19 are used to create the following five groups. They are Group 1 consisting of the image 1 and images 2 through 5 of primary differentiation; Group 2 containing the image 1 and images 6 through 9 of secondary differentiation; Group 3 including the image 1 and images 10 and 11 based on curvature; Group 4 including the image 1 and images 12 and 13 using the statistical quantity; and Group 5 including the image 1 and wavelet-based images 14 through 19. In this case, the image 1 as an original image is included repeatedly in all the groups.

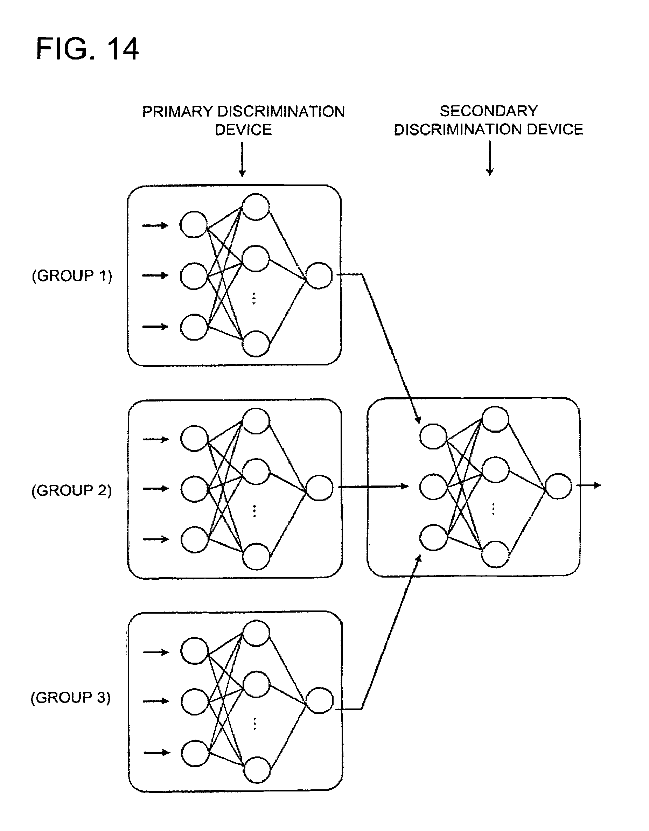

Learning of the discrimination device is carried out according to these groups (hereinafter abbreviated as "group learning"). In this case, as shown in Fig. 14, a separate discrimination device (primary discrimination device) is prepared for each group. A hierarchical group of discrimination devices is formed in such a way that the output value coming from the primary discrimination device of each group is further inputted into the secondary discrimination device and a comprehensive output value is obtained. After that, learning of the discrimination device is performed in two stages. The following describes this embodiment. In the first place, learning of each of the primary discrimination devices is conducted using the training input image of each group. In this case, the output value obtained from the primary discrimination device is compared with the pixel value of the training output image, and learning is performed. The same image as the aforementioned training input image is applied, as the image to be processed, to each of the primary discrimination devices having learnt, whereby the primary enhanced image is formed. This is followed by the step of learning of the secondary discrimination device, wherein the five created primary enhanced images are used as the training input images. In this case, learning is conducted through comparison between the output value obtained from the secondary discrimination device and the pixel value of the training output image. In Fig. 14, the ANN is used as an example for both the primary and secondary discrimination devices. The discrimination devices based on different techniques (ANN for the primary discrimination device, and discrimination/analysis method for the secondary discrimination device for example) can be used.

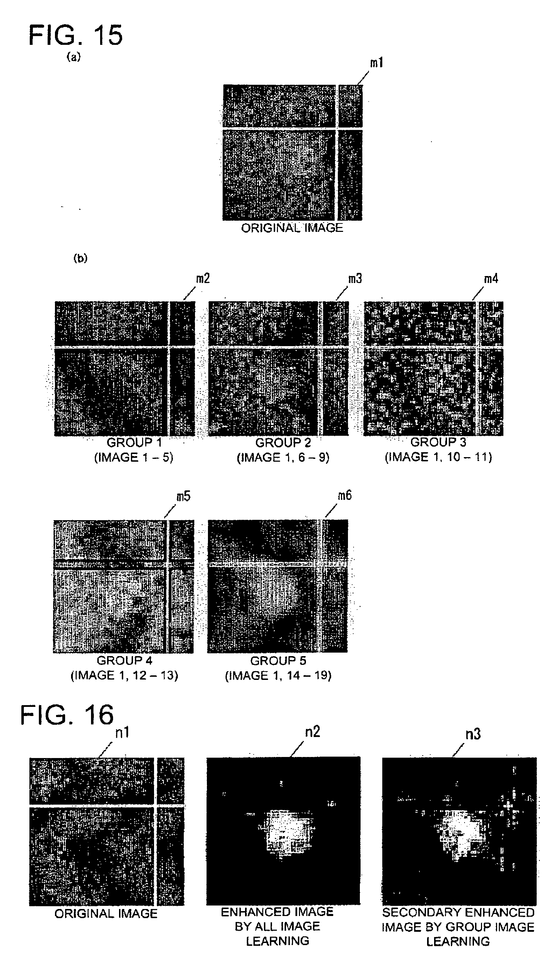

For example, the training feature images 2 through 19 of Fig. 3 are created from the original image m1 shown in Fig. 15 (a), and images 1 through 19 are classified into the aforementioned five groups. After that, group learning of the primary and secondary discrimination devices is performed. In this case, the original image m1 is inputted into the discrimination device having learnt. Then primary enhanced images m2 through m6 shown in Fig. 15 (b) are outputted from the primary discrimination device of each group. As shown in Fig. 15 (b), mutually different features are enhanced in the primary enhanced images m2 through m6. When these primary enhanced images m2 through m6 is further inputted into the secondary discrimination device, the secondary enhanced image n3 shown in Fig. 16 is obtained.

For the sake of comparison, Fig. 16 shows the secondary enhanced image n3, original image n1, and the enhanced image n2 wherein learning of all the images is achieved by one discrimination device, without being classified into groups (learning by the method of the present embodiment; hereinafter abbreviated as "all image learning"). As shown in Fig. 16, the secondary enhanced image n3 obtained by group learning has the features different from those of the enhanced image n2 resulting from all image learning. Fig. 16 shows the result of learning a simple circular pattern. In the case of group learning, as the patterns to be learnt get more and more complicated, the sensitivity to the pattern is improved, and the effect of group learning is expected to be enhanced.

In another embodiment, one discrimination device can be used as shown in Fig. 5, and learning of the discrimination device 20 can be conducted conforming to the aforementioned group. To put it more specifically, restrictions are placed to the coefficient of bondage between the neurons of the input and intermediate layers so as to relatively reduce the bondage of specific combinations or to block the bondage. For example, restrictions are imposed in such a way as to reduce the bondage between the neuron 1 on the intermediate layer and the neuron of the input layer corresponding to the training feature image which does not belong to the group 1. Restrictions are also put in such a way as to reduce the bondage between the neuron 2 on the intermediate layer and the neuron of the input layer corresponding to the training feature image which does not belong to the group 2. Learning of the discrimination device 20 is performed under these conditions.

As described above, use of a discrimination device conforming to each group makes it possible to implement a discrimination device 20 having a high degree of sensitivity to a specific pattern to be detected. It is also possible to provide an enhanced image having higher pattern recognition capability to a specific pattern. To put it another way, this arrangement permits flexible designing of a discrimination device conforming to the purpose of use characterized by a high degree of freedom, and therefore, ensures extremely practical use. Further, when the image having a relatively complicated pattern is to be processed, excellent advantages can be expected as well.

Various forms of image processing can be considered to create a training feature image. A discrimination device 20 designed specifically for a particular pattern can be obtained by selecting the form of image processing that appears effective for pattern enhancement, based on the feature of the pattern to be enhanced, or by selecting in such a way as to include the combination between the image processing that is effective for pattern enhancement and image processing that is effective for pattern reduction. It is also possible to select the feature image in conformity to the pattern to be enhanced, from among a great number of training feature images, using the optimization method such as a sequential selection method or genetic algorithm.

## Claims

1. An image processing method comprising:
making a discrimination device capable of pattern recognition by pattern learning based on training data learn a specific pattern in a learning step by using training images which have the specific pattern and comprise a training input image whose data is to be inputted into the discrimination device for learning of the discrimination device and a training output image having been prepared as a target of an image to be obtained after the learning of the discrimination device and corresponding to the training input image;
wherein the learning step comprises:
inputting data of the training input image into the discrimination device;
setting data of the training output image in the discrimination device; and
making the discrimination device learn so as to reduce an error between the data of the training output image and an output value from the discrimination device; and
wherein the image processing method further comprises:
creating an enhanced image, on which the specific pattern has been enhanced, from an image to be processed by using the discrimination device in an enhancing step by inputting data of the image to be processed into the discrimination device after the learning step;
and wherein each training input image is made of an original image which has the specific pattern and a plurality of training input images which are images created by applying different image processing to the original image, and
in the learning step, the pixel value of a pixel of interest located at the corresponding position in the original image and the pixel values of the pixel of interest located at the corresponding position in each of the plurality of training input images are inputted into the discrimination device, and in the training output image, a pixel value of a pixel corresponding to the pixel of interest is used as a learning target value of the discrimination device for the inputted values.

2. The image processing method described in claim 1, wherein, in the enhancing step, a plurality of feature images are created by applying different image processing to the image to be processed, and a pixel value of a pixel of interest located at a corresponding position in each of images to be processed including the plurality of feature images is inputted into the discrimination device, and an enhanced image is structured in such a way that an output value outputted based on the inputted value by the discrimination device is used as a pixel value of a pixel corresponding to the pixel of interest.

3. The image processing method described in any one of claims 1 or 2, wherein the training output image is an image created by processing the training input image.

4. The image processing method described in any one of claims 1 through 3, wherein the training output image is pattern data formed by converting the specific pattern into a function.

5. The image processing method described in claim 3 or 4, wherein the pixel value of the training output image is an indiscrete value.

6. The image processing method described in claim 3 or 4, wherein the pixel value of the training output image is a discrete value.

7. The image processing method described in any one of claims 1 or 2, wherein, in the learning step, the training feature images are grouped according to a characteristic of the image processing applied for the training feature image, and the discrimination device learns according to the group.

8. The image processing method described in any one of claims 1 through 7, wherein the training image is a medical image.

9. The image processing method described in claim 8, wherein the training image is a partial image formed by partial extraction from the medical image.

10. The image processing method described in claim 8 or 9, wherein the specific pattern indicates an abnormal shadow.

11. The image processing method described in any one of claims 1 through 10, further comprising: detecting an abnormal shadow candidate by using the enhanced image.

12. An image processing apparatus comprising:
a discrimination device which is capable of pattern recognition by pattern learning based on training data and outputs an output value after the recognition;
a learning device for making a discrimination device learn a specific pattern by using a training images which have the specific pattern and comprise a training input image whose data is to be inputted into the discrimination device for learning of the discrimination device and a training output image having been prepared as a target of an image to be obtained after the learning of the discrimination device and corresponding to the training input image;
wherein the learning device is configured to perform a learning step comprising inputting data of the training input image into the discrimination device, setting data of the training output image in the discrimination device, and making the discrimination device learn so as to reduce an error between the data of the training output image and an output value from the discrimination device; and
an enhancing device for creating an enhanced image, on which the specific pattern has been enhanced, from an image to be processed by inputting data of the image to be processed into the discrimination device after the learning by the learning device;
and wherein each training input image is made of an original image which has the specific pattern and a plurality of training input images which are images created by applying different image processing to the original image, and
the learning device is configured so that in the learning step, the pixel value of a pixel of interest located at the corresponding position in the original image and the pixel values of the pixel of interest located at the corresponding position in each of the plurality of training input images are inputted into the discrimination device, and in the training output image, a pixel value of a pixel corresponding to the pixel of interest is used as a learning target value of the discrimination device for the inputted values.

13. The image processing apparatus described in claim 12, wherein the enhancing device creates a plurality of feature images by application of different image processing to the image to be processed, and inputs a pixel value of a pixel of interest located at a corresponding position in each of images to be processed including the plurality of the feature images into the discrimination device, and structures an enhanced image in such a way that an output value outputted based on the inputted value by the discrimination device is used as a pixel value of a pixel corresponding to the pixel of interest.

14. The image processing apparatus described in any one of claims 12 or 13, wherein the training output image is an image created by processing the training input image.

15. The image processing apparatus described in any one of claims 12 or 13, wherein the training output image is a pattern data formed by converting the specific pattern included in the training input image into a function.

16. The image processing apparatus described in claims 14 or 15, wherein the pixel value of the training output image is an indiscrete value.

17. The image processing apparatus described in claim 14 or 15, wherein the pixel value of the training output image is an discrete value.

18. The image processing apparatus described in any one of claims 12 through 13, wherein the learning device groups the training feature images according to a characteristic of the image processing applied for the training feature image, and the discrimination device learns according to the group.

19. The image processing apparatus described in any one of claims 12 through 18, wherein the training image is a medical image.

20. The image processing apparatus described in claim 19, wherein the training image is a partial image formed by partial extraction from the medical image.

21. The image processing apparatus described in claim 19 or 20, wherein the specific pattern indicates an abnormal shadow.

22. The image processing apparatus described in any one of claims 12 through 21, further comprising: an abnormal shadow candidate detecting device for detecting an abnormal shadow candidate by using the enhanced image.

## Patentansprüche

1. Bildbearbeitungsverfahren mit:
Veranlassen einer Entscheidungsvorrichtung, die mittels Musterlernen beruhend auf Trainingsdaten zur Mustererkennung in der Lage ist, ein bestimmtes Muster in einem Lernschritt zu lernen, wobei Trainingsbilder, die das spezifische Muster haben, und ein Trainingseingabebild, dessen Daten der Entscheidungsvorrichtung für das Lernen der Entscheidungsvorrichtung vorzugeben sind, und ein Trainingsausgabebild, das als ein Ziel eines Bildes vorbereitet wurde, das nach dem Lernen durch die Entscheidungsvorrichtung zu Erhalten ist und dem Trainingseingabebild entspricht, verwendet werden;
wobei der Lernschritt umfasst:
Eingeben von Daten des Trainingseingabebildes in die Entscheidungsvorrichtung;
Einstellen von Daten des Trainingsausgabebildes in der Entscheidungsvorrichtung;
Veranlassen der Entscheidungsvorrichtung zum Lernen, um so einen Fehler zwischen den Daten des Trainingsausgabebildes und eines Ausgabewertes von der Entscheidungsvorrichtung zu verringern;
wobei das Bildbearbeitungsverfahren des Weiteren umfasst:
Erzeugen eines vergrößerten Bildes, bei dem das spezifische Muster vergrößert wurde, aus einem Bild, das mit der Entscheidungsvorrichtung zu verarbeiten ist, in einem Vergrößerungsschritt, in dem Daten des zu verarbeitenden Bildes in die Entscheidungsvorrichtung nach dem Lernschritt eingegeben werden;
und wobei jedes Trainingseingabebild aus einem Originalbild, das das spezifische Muster hat, und einer Mehrzahl von Trainingseingabebildern gemacht ist, die Bilder sind, welche durch Anwenden verschiedener Bildbearbeitungen auf das Originalbild erzeugt werden, und
wobei in dem Lernschritt der Pixelwert eines interessierenden Pixels, das an einer entsprechenden Position in dem Originalbild angeordnet ist, und die Pixelwerte der interessierenden Pixel, die an der entsprechenden Position in jedem der Mehrzahl von Trainingseingabebildern angeordnet sind, in die Entscheidungsvorrichtung eingegeben werden, und wobei in dem Trainingsausgabebild ein Pixelwert eines Pixels entsprechend dem interessierendem Pixel als Lernzielwert für die Entscheidungsvorrichtung aus den eingegebenen Werten verwendet wird.

2. Bildbearbeitungsverfahren nach Anspruch 1, bei dem in dem Vergrößerungsschritt eine Mehrzahl von Merkmalsbildern durch Anwenden von unterschiedlichen Bildbearbeitungen auf das zu verarbeitende Bild erzeugt werden, und wobei ein Pixelwert eines interessierenden Pixels an einer entsprechenden Position in jedem der zu verarbeitenden Bilder angeordnet ist, einschließlich der Mehrzahl von Merkmalsbildern, in die Entscheidungsvorrichtung eingegeben wird, und wobei ein vergrößertes Bild in einer solchen Art strukturiert ist, dass ein Ausgabewert, der beruhend auf dem eingegebenen Wert von der Entscheidungsvorrichtung ausgegeben wird, als Pixelwert eines Pixels entsprechend dem interessierendem Pixel.

3. Bildbearbeitungsverfahren nach einem der Ansprüche 1 oder 2, bei dem das Trainingsausgabebild ein Bild ist, das durch Verarbeiten des Trainingseingabebildes erhalten wird.

4. Bildbearbeitungsverfahren nach einem der Ansprüche 1 bis 3, bei dem das Trainingsausgabebild Musterdaten sind, die durch Umwandeln des spezifischen Musters in eine Funktion gebildet werden.

5. Bildbearbeitungsverfahren nach einem der Ansprüche 3 oder 4, bei dem der Pixelwert des Trainingsausgabebildes ein nicht-diskreter Wert ist.

6. Bildbearbeitungsverfahren nach einem der Ansprüche 3 oder 4, bei dem der Pixelwert des Trainingsausgabebildes ein diskreter Wert ist.

7. Bildbearbeitungsverfahren nach einem der Ansprüche 1 oder 2, bei dem in dem Lernschritt die Trainingsmerkmalsbilder entsprechend einer Charakteristik der Bildbearbeitung gruppiert werden, die auf das Trainingsmerkmalbild angewendet werden, und wobei die Entscheidungsvorrichtung entsprechend der Gruppe lernt.

8. Bildbearbeitungsverfahren nach einem der Ansprüche 1 bis 7, bei dem das Trainingsausgabebild ein medizinisches Bild ist.

9. Bildbearbeitungsverfahren nach Anspruch 8, bei denen das Trainingsausgabebild ein Teilbild durch Teilextraktion aus einem medizinischen Bild ist.

10. Bildbearbeitungsverfahren nach Anspruch 8 oder 9, bei dem das spezifische Muster einen anormalen Schatten angibt.

11. Bildbearbeitungsverfahren nach einem der Ansprüche 1 bis 10 des Weiteren mit:
Erfassen eines anormalen Schattenkandidaten unter Verwendung eines vergrößerten Bildes.

12. Bildbearbeitungsvorrichtung mit:
einer Entscheidungsvorrichtung, die in der Lage ist, eine Mustererkennung durchzuführen, in dem Muster beruhend auf Trainingsdaten gelernt werden, und die einen Ausgabewert nach dem Erkennen ausgibt;
einer Lernvorrichtung zum Veranlassen einer Entscheidungsvorrichtung zum Lernen eines spezifischen Musters unter Verwendung von Trainingsbildern, die das spezifische Muster enthalten, und eines Trainingseingabebildes, dessen Daten in die Entscheidungsvorrichtung eingegeben werden, um durch die Entscheidungsvorrichtung zu lernen, und eines Trainingsausgabebildes, das als Ziel eines Bildes vorbereitet wurde, das nach dem Lernen durch die Entscheidungsvorrichtung zu erhalten ist und dem Trainingseingabebild entspricht;
wobei die Lernvorrichtung ausgestaltet ist, um einen Lernschritt durchzuführen, der das Eingeben von Daten des Trainingseingabebildes in die Entscheidungsvorrichtung, das Einstellen der Daten des Trainingsausgabebildes in der Entscheidungsvorrichtung und das Durchführen durch die Entscheidungsvorrichtung des Lernens umfasst, um so einen Fehler zwischen den Daten des Trainingsausgabebildes und eines Ausgabewertes von der Entscheidungsvorrichtung zu verringern;
einer Vergrößerungsvorrichtung zum Erzeugen eines vergrößerten Bildes, auf dem das spezifische Muster vergrößert ist, aus einem zu verarbeitenden Bild durch Eingeben von Daten des zu verarbeitenden Bildes in die Entscheidungsvorrichtung nach dem Lernen durch die Lernvorrichtung;
und wobei jedes Trainingseingabebild aus einem Originalbild gemacht ist, das ein spezifisches Muster hat, und aus einer Mehrzahl von Trainingseingabebildern, die Bilder sind, die durch Anwenden verschiedener Bildbearbeitung auf das Originalbild erzeugt werden, und
wobei die Lernvorrichtung ausgestaltet ist, so dass bei dem Lernschritt der Pixelwert eines interessierenden Pixels, das an einer entsprechenden Stelle in dem Originalbild angeordnet ist, und die Pixelwerte der interessierenden Pixels, die an der entsprechenden Position in jedem der Mehrzahl von Trainingseingabebildern positioniert sind, in die Entscheidungsvorrichtung eingegeben werden, und wobei in dem Trainingsausgabebild ein Pixelwert eines Pixels entsprechend dem interessierendem Pixel als Lernzielwert der Entscheidungsvorrichtung für die eingegebenen Werte verwendet wird.

13. Bildbearbeitungsvorrichtung nach Anspruch 12, bei der die Vergrößerungsvorrichtung eine Mehrzahl von Merkmalsbildern erzeugt, wobei verschiedene Bildbearbeitungen an das zu bearbeitende Bild angewendet werden, und die einen Pixelwert eines interessierenden Pixels in die Entscheidungsvorrichtung eingibt, das an einer entsprechenden Position in jedem der zu bearbeitenden Bilder angeordnet ist, einschließlich der Mehrzahl von Merkmalsbildern, und die ein vergrößertes Bild in einer solchen Art strukturiert, dass ein Ausgabewert, der beruhend auf dem eingegebenen Wert durch die Entscheidungsvorrichtung ausgegeben wird, als ein Pixelwert eines Pixels entsprechend dem interessierendem Pixel verwendet wird.

14. Bildbearbeitungsvorrichtung nach einem der Ansprüche 12 oder 13, bei dem das Trainingsausgabebild ein Bild ist, das beim Bearbeiten des Trainingseingabebildes erzeugt wird.

15. Bildbearbeitungsvorrichtung nach einem der Ansprüche 12 oder 13, bei dem das Trainingsausgabebild Musterdaten sind, die durch Umwandeln des spezifischen Musters, das in dem Trainingseingabebild enthalten ist, in eine Funktion gebildet werden.

16. Bildbearbeitungsvorrichtung nach Anspruch 14 oder 15, bei der der Pixelwert des Trainingsausgabebildes ein nicht-diskreter Wert ist.

17. Bildbearbeitungsvorrichtung nach Anspruch 14 oder 15 bei der der Pixelwert des Trainingsausgabebildes ein diskreter Wert ist.

18. Bildbearbeitungsvorrichtung nach einem der Ansprüche 12 bis 13, bei der die Lernvorrichtung die Trainingsmerkmalsbilder entsprechend einer Charakteristik der auf das Trainingsmerkmalsbild angewandten Bildbearbeitung gruppiert, und wobei die Entscheidungsvorrichtung entsprechend der Gruppe lernt.

19. Bildbearbeitungsvorrichtung nach einem der Ansprüche 12 bis 18, bei der das Trainingsausgabebild ein medizinisches Bild ist.

20. Bildbearbeitungsvorrichtung nach Anspruch 19, bei der das Trainingsausgabebild ein Teilbild ist, das durch Teilextraktion aus einem medizinischen Bild gebildet wird.

21. Bildbearbeitungsvorrichtung nach Anspruch 19 oder 20, bei der das spezifische Muster einen anormalen Schatten anzeigt.

22. Bildbearbeitungsvorrichtung nach einem der Ansprüche 12 bis 21, des Weiteren mit einer anormal Schattenkandidaten-Selektionsvorrichtung zum Erfassen eines anormalen Schattenkandidaten unter Verwendung des vergrößerten Bildes.

## Revendications

1. Procédé de traitement d'image comprenant l'étape consistant à :
amener un dispositif de discrimination capable de reconnaître un motif par un apprentissage de motif basé sur des données d'apprentissage à apprendre un motif spécifique à une étape d'apprentissage en utilisant des images d'apprentissage qui comportent le motif spécifique et qui comprennent une image d'entrée d'apprentissage dont les données doivent être entrées dans le dispositif de discrimination pour l'apprentissage du dispositif de discrimination et une image de sortie d'apprentissage qui a été préparée en tant que cible d'une image à obtenir après l'apprentissage du dispositif de discrimination et correspondant à l'image d'entrée d'apprentissage ;
dans lequel l'étape d'apprentissage comprend les étapes consistant à :
entrer les données de l'image d'entrée d'apprentissage dans le dispositif de discrimination ;
configurer les données de l'image de sortie d'apprentissage dans le dispositif de discrimination ; et
amener le dispositif de discrimination à apprendre de manière à réduire une erreur entre les données de l'image de sortie d'apprentissage et une valeur de sortie du dispositif de discrimination ; et
dans lequel le procédé de traitement d'image comprend en outre les étapes consistant à :
créer une image améliorée, sur laquelle le motif spécifique a été amélioré, à partir d'une image à traiter en utilisant le dispositif de discrimination à une étape d'amélioration en entrant les données de l'image à traiter dans le dispositif de discrimination après l'étape d'apprentissage ;
et dans lequel chaque image d'entrée d'apprentissage est constituée d'une image originale qui comporte le motif spécifique et d'une pluralité d'images d'entrée d'apprentissage qui sont des images créées en appliquant un traitement d'image différent à l'image originale, et
à l'étape d'apprentissage, la valeur de pixel d'un pixel d'intérêt situé à la position correspondante dans l'image originale et les valeurs de pixel du pixel d'intérêt situé à la position correspondante dans chacune de la pluralité d'images d'entrée d'apprentissage sont entrées dans le dispositif de discrimination, et dans l'image de sortie d'apprentissage, une valeur de pixel d'un pixel correspondant au pixel d'intérêt est utilisée en tant que valeur cible d'apprentissage du dispositif de discrimination pour les valeurs entrées.

2. Procédé de traitement d'image selon la revendication 1, dans lequel, à l'étape d'amélioration, une pluralité d'images de caractéristique sont créées en appliquant un traitement d'image différent à l'image à traiter, et une valeur de pixel d'un pixel d'intérêt situé à une position correspondante dans chacune des images à traiter comprenant la pluralité d'images de caractéristique est entrée dans le dispositif de discrimination, et une image améliorée est structurée de manière à ce qu'une valeur de sortie qui est sortie sur la base de la valeur entrée par le dispositif de discrimination soit utilisée en tant que valeur de pixel d'un pixel correspondant au pixel d'intérêt.

3. Procédé de traitement d'image selon l'une quelconque des revendications 1 et 2, dans lequel l'image de sortie d'apprentissage est une image créée en traitant l'image d'entrée d'apprentissage.

4. Procédé de traitement d'image selon l'une quelconque des revendications 1 à 3, dans lequel l'image de sortie d'apprentissage consiste en des données de motif formées en convertissant le motif spécifique en une fonction.

5. Procédé de traitement d'image selon la revendication 3 ou 4, dans lequel la valeur de pixel de l'image de sortie d'apprentissage est une valeur non discrète.

6. Procédé de traitement d'image selon la revendication 3 ou 4, dans lequel la valeur de pixel de l'image de sortie d'apprentissage est une valeur discrète.

7. Procédé de traitement d'image selon l'une quelconque des revendications 1 et 2, dans lequel, à l'étape d'apprentissage, les images de caractéristique d'apprentissage sont groupées conformément à une caractéristique du traitement d'image appliqué pour l'image de caractéristique d'apprentissage, et le dispositif de discrimination apprend conformément au groupe.

8. Procédé de traitement d'image selon l'une quelconque des revendications 1 à 7, dans lequel l'image d'apprentissage est une image médicale.

9. Procédé de traitement d'image selon la revendication 8, dans lequel l'image d'apprentissage est une image partielle formée par extraction partielle à partir de l'image médicale.

10. Procédé de traitement d'image selon la revendication 8 ou 9, dans lequel le motif spécifique indique une ombre anormale.

11. Procédé de traitement d'image selon l'une quelconque des revendications 1 à 10, comprenant en outre : la détection d'une ombre anormale candidate en utilisant l'image améliorée.

12. Appareil de traitement d'image comprenant :
un dispositif de discrimination qui est capable de reconnaître un motif par un apprentissage de motif sur la base de données d'apprentissage et qui délivre une valeur de sortie après la reconnaissance ;
un dispositif d'apprentissage pour amener un dispositif de discrimination à apprendre un motif spécifique en utilisant des images d'apprentissage qui comportent le motif spécifique et qui comprennent une image d'entrée d'apprentissage dont les données doivent être entrées dans le dispositif de discrimination pour l'apprentissage du dispositif de discrimination et une image de sortie d'apprentissage qui a été préparée en tant que cible d'une image à obtenir après l'apprentissage du dispositif de discrimination et correspondant à l'image d'entrée d'apprentissage ;
dans lequel le dispositif d'apprentissage est configuré pour effectuer une étape d'apprentissage comprenant les étapes consistant à entrer les données de l'image d'entrée d'apprentissage dans le dispositif de discrimination, configurer les données de l'image de sortie d'apprentissage dans le dispositif de discrimination, et amener le dispositif de discrimination à apprendre de manière à réduire une erreur entre les données de l'image de sortie d'apprentissage et une valeur de sortie du dispositif de discrimination ; et
un dispositif d'amélioration pour créer une image améliorée, sur laquelle le motif spécifique a été amélioré, à partir d'une image à traiter en entrant les données de l'image à traiter dans le dispositif de discrimination après l'apprentissage par le dispositif d'apprentissage ;
et dans lequel chaque image d'entrée d'apprentissage est constituée d'une image originale qui comporte le motif spécifique et d'une pluralité d'images d'entrée d'apprentissage qui sont des images créées en appliquant un traitement d'image différent à l'image originale, et
le dispositif d'apprentissage est configuré de sorte que, à l'étape d'apprentissage, la valeur de pixel d'un pixel d'intérêt situé à la position correspondante dans l'image originale et les valeurs de pixel du pixel d'intérêt situé à la position correspondante dans chacune de la pluralité d'images d'entrée d'apprentissage sont entrées dans le dispositif de discrimination, et dans l'image de sortie d'apprentissage, une valeur de pixel d'un pixel correspondant au pixel d'intérêt est utilisée en tant que valeur cible d'apprentissage du dispositif de discrimination pour les valeurs entrées.

13. Appareil de traitement d'image selon la revendication 12, dans lequel le dispositif d'amélioration crée une pluralité d'images de caractéristique par l'application d'un traitement d'image différent à l'image à traiter, et entre une valeur de pixel d'un pixel d'intérêt situé à une position correspondante dans chacune des images à traiter comprenant la pluralité des images de caractéristique dans le dispositif de discrimination, et structure une image améliorée de manière à ce qu'une valeur de sortie qui est sortie sur la base de la valeur entrée par le dispositif de discrimination soit utilisée en tant que valeur de pixel d'un pixel correspondant au pixel d'intérêt.

14. Appareil de traitement d'image selon l'une quelconque des revendications 12 et 13, dans lequel l'image de sortie d'apprentissage est une image créée en traitant l'image d'entrée d'apprentissage.

15. Appareil de traitement d'image selon l'une quelconque des revendications 12 et 13, dans lequel l'image de sortie d'apprentissage consiste en des données de motif formées en convertissant le motif spécifique inclus dans l'image d'entrée d'apprentissage en une fonction.

16. Appareil de traitement d'image selon la revendication 14 ou 15, dans lequel la valeur de pixel de l'image de sortie d'apprentissage est une valeur non discrète.

17. Appareil de traitement d'image selon la revendication 14 ou 15, dans lequel la valeur de pixel de l'image de sortie d'apprentissage est une valeur discrète.

18. Appareil de traitement d'image selon l'une quelconque des revendications 12 et 13, dans lequel le dispositif d'apprentissage groupe les images de caractéristique d'apprentissage conformément à une caractéristique du traitement d'image appliqué pour l'image de caractéristique d'apprentissage, et le dispositif de discrimination apprend conformément au groupe.

19. Appareil de traitement d'image selon l'une quelconque des revendications 12 à 18, dans lequel l'image d'apprentissage est une image médicale.

20. Appareil de traitement d'image selon la revendication 19, dans lequel l'image d'apprentissage est une image partielle formée par extraction partielle à partir de l'image médicale.

21. Appareil de traitement d'image selon la revendication 19 ou 20, dans lequel le motif spécifique indique une ombre anormale.

22. Appareil de traitement d'image selon l'une quelconque des revendications 12 à 21, comprenant en outre : un dispositif de détection d'ombre anormale candidate pour détecter une ombre anormale candidate en utilisant l'image améliorée.
